# EUROPEAN PATENT APPLICATION

(11) **EP 4 295 891 A1**
(43) Date of publication of application: **27.12.2023**
(21) Application number: 22755493.8
(22) Date of filing: 24.01.2022
(51) Int. Cl.: A61M 25/10, A61M 25/00, A61L 29/16, A61L 29/00

(54) **DRUG-LOADED MEDICAL INSTRUMENT AND PREPARATION METHOD THEREFOR**

(30) Priority: 22.02.2021 CN 202110195532
(71) Applicant: Shanghai MicroPort Medical (Group) Co., Ltd., Shanghai 201203 (CN)
(72) Inventor: LI, Meng, Shanghai 201203 (CN); DU, Qiran, Shanghai 201203 (CN); LI, Junfei, Shanghai 201203 (CN)
(74) Representative: Marks & Clerk LLP
(86) International application number: PCT/CN2022/073345
(87) International publication number: WO 2022/174720

(57) **Abstract**

The present application relates to the field of pharmaceutical technology, in particular, to a drug-loaded medical device, including a medical device body and at least one drug coating, the drug coating is attached to at least one side of a surface of the medical device body. The main component of the drug coating includes a macrolide drug. The drug coating includes elongated prismatic crystals each with at least three lateral edges, and/or the drug coating includes crystal clusters of the elongated prismatic crystals each with at least three lateral edges. An aspect ratio of each elongated prismatic crystal is in a range from 1: 1 to 40: 1. The drug-loaded medical device can effectively improve the drug transfer to the blood vessel wall and prolong the concentration of drug in tissue.

## Description

### CROSS-REFERENCE TO RELATED APPLICATIONS

This application claims priority to Chinese patent application No. 202110195532.5, titled "DRUG-LOADED MEDICAL INSTRUMENT AND PREPARATION METHOD THEREFOR", filed on February 22, 2021, in the China National Intellectual Property Administration, the content of which is hereby incorporated by reference in its entirety.

### TECHNICAL FIELD

The present application relates to the technical field of medical devices, in particular, to a drug-loaded medical device and a preparation method thereof.

### BACKGROUND

In recent years, a variety of interventional or implantable medical devices containing drug coating have been developed for various mechanisms of vascular restenosis. The drug-coated balloons and the drug eluting stents both essentially stem from the concept of catheter-based devices for local drug delivery. They achieve the inhibition of intimal hyperplasia by carrying drugs, albeit differing in the method of drug delivery and the duration of local drug action. The drug-coated balloons have attracted more and more attention due to the concept of "intervention without implantation". Numerous clinical trials have already demonstrated satisfactory efficacy and safety of the drug-coated balloons in various conditions such as coronary artery stenosis, small vessel diseases, and bifurcation lesions. The drug-coated balloons are uniformly coated with anti-proliferative drugs, and after delivery to the lesion site, the drugs are released within a short expansion time (30s to 60s), inhibiting the proliferation of vascular smooth muscle cells.

Recently, it has been found that the drug formulation used in implantable or interventional drug-loaded medical devices plays a crucial role in drug transfer and retention. It has been discovered that drugs in crystalline form have better transfer and retention performances compared to drugs in amorphous form. The crystalline drugs have higher drug concentrations in tissue over an extended period, and the effective drug concentrations can be maintained for more than 28 days, which is particularly important for inhibiting restenosis. However, it is challenging to obtain crystalline macrolide structures using traditional methods such as ultrasound spraying or drop coating. These methods often result in a drug in an amorphous state. Even though the crystallization is promoted by the method such as adjusting the supersaturation of macrolide drugs, the resulting crystalline particles often exhibit rhombic or spheroidal morphologies. The crystalline drugs with these rounded morphologies have weak interactions with the vascular wall, leading to unsatisfactory drug transfer performance. Consequently, the preparation of elongated rod-shaped crystalline particles with sharp morphologies for macrolide drugs is highly challenging.

### SUMMARY

The present application relates to a drug-loaded medical device, including a medical device body and at least one drug coating, the drug coating is attached to at least one side of a surface of the medical device body.

A main component of the drug coating includes a macrolide drug. The drug coating includes elongated prismatic crystals each with at least three lateral edges, and/or the drug coating includes crystal clusters of the elongated prismatic crystals each with at least three lateral edges. An aspect ratio of each elongated prismatic crystal is in a range from 1: 1 to 40: 1.

The elongated prismatic crystal includes at least two adjacent lateral surfaces with an included angle smaller than 90 degrees.

The macrolide drug includes at least one of rapamycin, zotarolimus, everolimus, temsirolimus, biolimus, 7-O-desmethylrapamycin, temsirolimus, ridaforolimus, 40-O-(2-hydroxyethyl)-rapamycin, 32-deoxo rapamycin, biolimus A9, ABT-578, SDZ RAD, or SAR943.

The present application also relates to a preparation method of a drug-loaded medical device, the main component of which includes the macrolide drug as defined above, the method including:
1) dispersing macrolide drug crystal powder in a solvent, adding rigid microspheres, agitating, and filtering out the rigid microspheres to obtain a suspension;
2) taking a medical device body including a solid-phase surface, applying the suspension onto the surface of the medical device body, and drying the surface to obtain a medical device intermediate; and
3) applying a supersaturated solution of the macrolide drug onto the surface of the medical device intermediate and drying the surface.

The beneficial effect of the present application is as follows:

The present application successfully prepares elongated prismatic active drug crystalline particles with high aspect ratio, sharp edges and corners, which can effectively improve the adhesion performance of the drug on the medical device, allowing prolonged delivery time of the medical device, and increase the transfer speed of the drug to a blood vessel wall, and prolong drug concentration in tissue.

### BRIEF DESCRIPTION OF THE DRAWINGS

In order to more clearly describe the technical solutions in the embodiments of the present application or the conventional art, the drawings to be used in the description of the embodiments or the conventional art will be described briefly. Apparently, the drawings described below are merely for some embodiments of the present application. For ordinary skilled persons in the art, other drawings can also be obtained based on the following drawings without creative work.

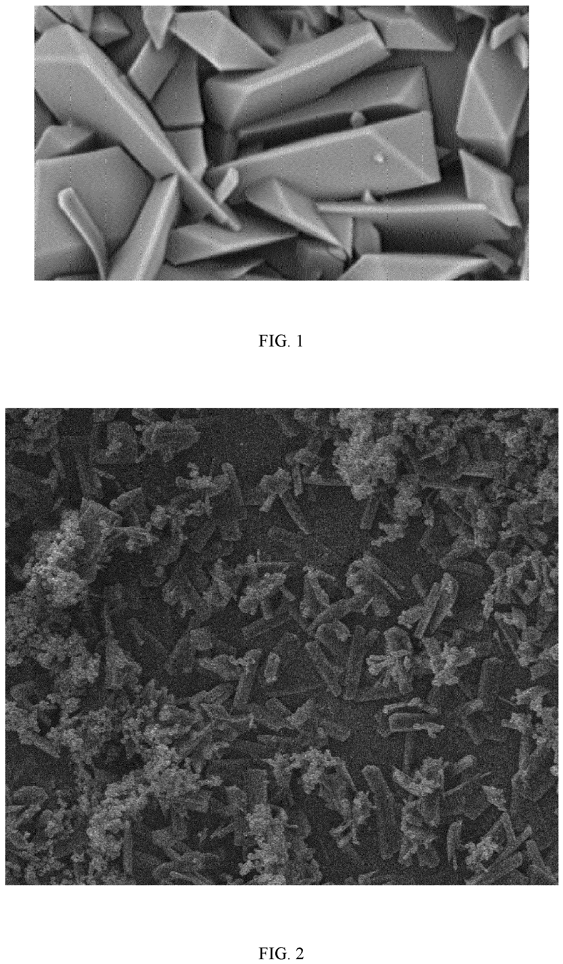

FIG. 1 shows a 5000 times magnified scanning electron micrograph of different positions of active drug crystalline particles prepared in Example 1.
FIG. 2 shows a 1000 times magnified scanning electron micrograph of active drug crystalline particles prepared in Example 4.
FIG. 3 shows a 5000 times magnified scanning electron micrograph of active drug crystalline particles prepared in Example 5.
FIG. 4 shows a 5000 times magnified scanning electron micrograph of active drug crystalline particles prepared in Example 11.
FIG. 5 shows a 2000 times magnified scanning electron micrograph of active drug crystalline particles prepared in Comparative Example 2.

### DETAILED DESCRIPTION

Reference will now be made in detail to embodiments of the present application, one or more examples of which are described below. Each example is provided by way of illustration, not limitation of the present application. It will be apparent to those skilled in the art that various modifications and variations can be made in the present application without departing from the scope or spirit of the present application. For example, features illustrated or described as part of one embodiment can be used in another embodiment to yield a further embodiment.

Unless otherwise defined, all technical and scientific terms used herein have the same meaning as commonly understood by one of ordinary skill in the art to which the present application belongs. The terms used herein in the specification of the present application are only for the purpose of describing specific embodiments, and are not intended to limit the present application. As used herein, the term "and/or" includes any and all combinations of one or more of the associated listed items.

The medical device mentioned in present application refers to any drug carrier used in a medical process, and is not limited to the definition of medical device in the medical industry.

The present application relates to a drug-loaded medical device, including a medical device body and at least one drug coating. The medical device includes a solid-phase surface, and the drug coating is attached to a side of the solid-phase surface. The drug coating includes elongated prismatic crystals each with at least three lateral edges, and/or the drug coating includes crystal clusters of the elongated prismatic crystals each with at least three lateral edges.

The crystals can be in at least two forms in the drug coating, one is elongated prisms, and the other is cluster aggregates or "crystal clusters". The crystal cluster includes a plurality of elongated prisms. The elongated prism includes at least three lateral edges. The cross-section of the elongated prism can be triangular or quadrangular, preferably rhomboidal.

The drug coating that includes the elongated prisms and/or crystal clusters has one or more of the following features:
a) The elongated prism is a solid structure with smooth surfaces and obvious edges and corners.
b) The upper base of the elongated prism can be one plane or multiple planes. The edges of the upper base of the elongated prism are substantially straight lines, and/or the lateral edges are substantially straight lines.
c) The upper and lower bases of the elongated prism can be parallel or non-parallel to each other.
d) The elongated prisms in the drug coating are not uniform in size, and two adjacent elongated prisms may be connected to each other or staggered.
e) There are gaps between the crystal clusters in the drug coating, and the growth direction of a single crystal cluster is consistent, with obvious edges and corners. The crystal clusters are inconsistent in size, and the shapes thereof are also slightly different.
f) In some cases, the drug coating surface presents individual elongated prisms in some areas while presents crystal clusters in other areas.
g) The maximum length of an elongated prism or a single crystal cluster is 25 µm, such as 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17µm, 18 µm, 19 µm, 20 µm, 21 µm, 22 µm, 23 µm, 24 µm, 25 µm. Preferably, the maximum length of an elongated prism or the maximum length of a single crystal cluster is 1-20 µm.
h) In some cases, the drug coating formed by the drug crystalline particles may include other compounds, such as other active drugs other than the macrolide drug. These other compounds are distributed in the gaps between the elongated prisms or the crystal clusters, or are attached to the surfaces of the elongated prisms or the crystal clusters.

In some embodiments, an aspect ratio of at least one elongated prismatic crystal in the drug coating is in a range from 1:1 to 40:1, such as 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 25:1, 30:1, 35:1.

Preferably, the aspect ratio of at least one elongated prismatic crystal in the drug coating is in a range from 2:1 to 20:1.

In some embodiments, an included angle between adjacent lateral surfaces of the elongated prism in the drug coating is smaller than 90 degrees, such as 80 degrees, 70 degrees, 60 degrees, 50 degrees, 40 degrees, 30 degrees, 20 degrees, 10 degrees, preferably smaller than 60 degrees, more preferably smaller than 40 degrees, further more preferably smaller than 35 degrees, and further more preferably between 20 degrees and 35 degrees.

In some embodiments, the elongated prismatic crystals and/or crystal clusters account for more than 90% by mass, preferably more than 98% by mass, of the drug coating on the surface of the medical device body.

In some embodiments, an included angle between the lateral edges of the elongated prismatic crystal and the surface of the medical device body is in a range from 0° to 90°, preferably 30° to 70°.

In some embodiments, the loss rate of the drug coating attached to the surface of the medical device body is less than or equal to 24%. The loss rate described in the present application refers to the loss rate of the drug during delivery of the drug-loaded medical device through a vascular model. The smaller the loss rate, the higher the utilization rate of the drug, giving the surgeon more operating time, and the surgeon's operation does not have to be restricted by the drug loss rate of the medical device, and does not need to be completed in a very short time.

In some embodiments, the loss rate of the drug coating attached to the surface of the medical device body is less than or equal to 20%.

In some embodiments, the loss rate of the drug coating attached to the surface of the medical device body is less than or equal to 15%.

In some embodiments, the thickness of the drug coating on the surface of the medical device body is in a range from 1 µm to 50 µm, such as 5 µm, 10 µm, 15 µm, 20 µm, 25 µm, 30 µm, 35 µm, 40 µm, 45 µm.

In some embodiments, the medical device body is a drug-coated balloon, a drug eluting stent, a medical patch, or a sheath for an introducer, etc.

In some embodiments, the macrolide drug is selected from the group consisting of a rapamycin drug, erythromycin, peplomycin, spiramycin, tilmicosin, azithromycin, clarithromycin (Biaxin), dirithromycin, roxithromycin, carbomycin A, josamycin, kitasamycin, oleandomycin, spiramycin, troleandomycin, tylosin, tacrolimus, telithromycin, cethromycin, and combinations thereof.

In some embodiments, the rapamycin drug is selected from the group consisting of rapamycin, zotarolimus, everolimus, temsirolimus, biolimus, 7-O-desmethylrapamycin, temsirolimus (CCI-779), ridaforolimus (or deforolimus), 40-O-(2-hydroxyethyl)-rapamycin (SDZ RAD), 32-deoxo rapamycin (SAR943), biolimus A9, ABT-578, SDZ RAD, SAR943, and combinations thereof.

In some embodiments, the macrolide drug accounts for 50% or above of the total mass of drug, such as 60%, 70%, 80%, 85%, 90%, 91%, 92%, 93%, 94%, 95%, 96%, 97%, 98%, 99%, 99.5%, 99.6%, 99.7 %, 99.8 %, 99.9%, or 100%.

In some embodiments, at least one elongated prism in the drug coating includes 4, 5, 6, 7, 8, 9, 10 or more lateral edges.

In some embodiments, the drug coating further includes at least one of a) to c) as follows:
a) a drug delivery carrier layer disposed on either the upper base or the lower base of the drug crystalline particles;
b) an antioxidant;
c) at least one active drug other than the macrolide drug.

In some embodiments, the main component of the drug delivery carrier layer includes at least one of calcium chloride, urea, polyvinylpyrrolidone, polyvinyl alcohol, collagen, gelatin, polysaccharide, stearic acid, iopromide, iopamidol, iohexol, ioversol, citric acid, glucan, polysorbate, sorbitol, chitosan, poloxamer, sodium carboxymethylcellulose, hydroxypropyl methylcellulose, sodium hyaluronate, sodium alginate, ammonium shellacate, n-butyryl tri-n-hexyl citrate (BTHC), shellac, or PEG.

In some embodiments, the antioxidant is selected from the group consisting of dibutyl hydroxytoluene, butylated hydroxyanisole, ascorbic acid, palmitate, tocopherol, probucol, vitamin E, polyethylene glycol succinate, and combinations thereof.

In some embodiments, the drug coating also includes an organic crystallization nucleating agent, selected from the group consisting of a salt of an organic acid, a dibenzylidene sorbitol derivative, a diamide, a hydrazide, and combinations thereof.

In some embodiments, the at least one active drug other than the macrolide drug is at least one of paclitaxel, a paclitaxel derivative, cilostazol, ticlopidine, a phosphodiesterase inhibitor, nafronyl, pentoxifylline, rivaroxaban, apixaban, dabigatran etexilate, tirofiban, a thrombin inhibitor, a factor Xa inhibitor, a vitamin K inhibitor, a cyclooxygenase inhibitor, an ADP receptor antagonist or inhibitor, a platelet glycoprotein IIb/IIIa receptor antagonist, dexamethasone, prednisolone, corticosterone, budesonide, a natriuretic peptide, an estrogen, sulfasalazine, aminosalicylic acid, acemetacin, aescin, aminopterin, antimycoin, arsenic trioxide, aristolochic acid, aspirin, berberine, bilobol, endostatin, angiostatin, angiopeptin, an monoclonal antibody that can block the proliferation of smooth muscle cells, levofloxacin, hydroxycamptothecin, vinblastine, vincristine, doxorubicin, 5-fluorouracil, cisplatin, a bisphosphonate, a selective estrogen receptor modulator, strontium ranelate, actinomycin D, cyclosporine A, cyclosporine C, brefeldin A, relaxin, a guanylate activating enzyme regulator, nitroxyl prodrug CXL-1020, a nitrate drug, sodium nitroprusside, nitroglycerin, a thymidine kinase inhibitor antibiotic, nesiritide, a calcium channel blocker such as diltiazem, verapamil, nicardipine, platelet vasodilator-stimulated phosphoprotein, vasodilator-stimulated phosphoprotein, papaverine, adenosine, magnesium sulfate, xantinol nicotinate, or nicorandil.

In some embodiments, the paclitaxel derivative includes one or more of docetaxel, nab-paclitaxel, or nap-paclitaxel.

The present application further relates to a preparation method of a drug-loaded medical device. The method includes following steps:
1) Prepare macrolide drug crystal powder and disperse it in a solvent, add rigid microspheres, agitate, and filter out the rigid microspheres to obtain a suspension.
2) Take a medical device body including a solid-phase surface, apply the suspension onto the surface of the medical device body, and dry the surface to obtain a medical device intermediate.
3) Apply a supersaturated solution of the macrolide drug onto the surface of the medical device intermediate and dry the surface.

In some embodiments, the suspension includes crystalline microparticles of the macrolide drug with a particle size in a range from 100 nm to 1000 nm.

In some embodiments, the particle size of the crystalline microparticles can be 200 nm, 300 nm, 400 nm, 500 nm, 600 nm, 700 nm, 800 nm, 900 nm, and further can be 200 nm to 500 nm.

In some embodiments, the purity of the macrolide drug in the crystalline microparticles is equal to or greater than 80%, and can be 85%, 90%, 91%, 92%, 93%, 94%, 95%, 96%, 97%, 98%, 99%, 99.5%, 99.6%, 99.7%, 99.8%, 99.9%, or 100%.

In some embodiments, the concentration of the supersaturated solution is in a range from 1 mg/mL to 100 mg/mL, and can be 2 mg/mL, 3 mg/mL, 4 mg/ mL, 5 mg/mL, 6 mg/mL, 7 mg/mL, 8 mg/mL, 9 mg/mL, 10 mg/mL, 11 mg/mL, 12 mg/mL, 13 mg/mL, 14 mg/mL, 15 mg /mL, 16 mg/mL, 17 mg/mL, 18 mg/mL, 19 mg/mL, 20 mg/mL, 30 mg/mL, 40 mg/mL, 50 mg/mL, 60 mg/mL, 70 mg/ mL, 80 mg/mL, 90 mg/mL; for example, 5 mg/mL to 70mg/mL, 10 mg/mL to 50mg/mL, or 20 mg/mL to 40mg/mL.

In some embodiments, the concentration of the macrolide drug in the solvent is in a range from 4 mg/10mL to 10 mg/10mL, and the mass of the rigid microspheres is in a range from 1g to 4g, the mixture is agitated on a vortex mixer for 8 min to 12 min, and the agitating speed is 2000rpm to 3000rpm.

In some embodiments, the solvent is an alkane, and can be at least one of n-heptane, octane, n-hexane, pentane, or cyclohexane.

In some embodiments, the rigid microspheres are made of a material including at least one of zirconium oxide, silicon nitride, hard stainless steel, hard tungsten carbide, sintered corundum, or agate. In some embodiments, the particle size of the rigid microspheres is 800 to 1200 times of the particle size of the crystalline microparticles, such as 900, 1000, or 1100 times. In some embodiments, the particle size of the rigid microspheres is in a range from 0.1 mm to 0.8 mm, preferably in a range from 0.2 mm to 0.5 mm.

In some embodiments, in step 2), a drug delivery carrier layer is disposed on the solid-phase surface of the carrier; in some embodiments, the drug delivery carrier is the drug delivery carrier as defined above.

The drug delivery carrier layer is coated on the surface of the implantable or interventional medical device, so that not only the subsequent coating of the supersaturated solution of the macrolide drug can be more uniform, but also the bioavailability of the drug can be improved. The drug delivery carrier layer plays the role of a stent and can increase contact between the drug coating and blood vessel wall, promoting the drug transfer. In some embodiments, step 2) further includes a step of spraying a solution of an organic crystallization nucleating agent onto the surface of the intermediate and drying it. The organic nucleating agent is selected from the group consisting of a salt of an organic acid, a dibenzylidene sorbitol derivative, a diamide, a hydrazide, and combinations thereof.

In some embodiments, the supersaturated solution of the macrolide drug further includes an antioxidant and/or at least one active drug other than the macrolide drug.

In some embodiments, the antioxidant is a pharmaceutical antioxidant as defined above; the antioxidant is preferably dibutylhydroxytoluene, which can modulate the crystallization behavior of macrolides.

In some embodiments, the at least one active drug other than the macrolide drug is at least one other active drug other than the macrolide drug as defined above.

In some embodiments, the method for coating is at least one of micropipetting, dip coating, brushing, or spraying (such as spraying with an ultrasonic piezoelectric nozzle).

The embodiments of the present application will be described in detail below in combination with examples.

In the following examples, the balloon catheter has a diameter of 3.0 mm and a length of 20 mm.

### Example 1

1 g of rapamycin raw material was dispersed in 50 mL of n-heptane, added with a stir bar, stirred overnight with a stirring speed of 300 rpm at room temperature under protection against light, and then filtered to obtain a filter cake. The filter cake was vacuum dried to obtain crystalline rapamycin, whose crystallinity was measured using infrared spectroscopy or powder X-ray diffraction, resulting that the crystallinity is above 90%.

7 mg of the above crystalline rapamycin was put in a brown bottle, added with 10 mL of n-heptane to disperse the rapamycin, and then added with 2.5 g of zirconia beads (Φ0.2-0.3 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto a surface of a balloon, and air dried for 2 hours under protection against light.

125 mg of rapamycin and 15 mg of dibutylhydroxytoluene were dissolved in a mixed solution of 3 mL of acetone and 2 mL of ethanol, and then added with 3 mL of purified water to form a supersaturated solution of rapamycin.

The prepared rapamycin supersaturated solution was uniformly sprayed by ultrasonic spray onto the surface of the balloon prepared in the previous step at a drug concentration of 3 µg/mm². The rotation speed of the balloon was 200 rpm, the moving speed of the ultrasonic spray nozzle was 0.2 mm/s, and the solution ejection speed was 0.8 µL/s. After air dried for 24 hours, the balloon surface was sterilized with ethylene oxide, and the morphology thereof was observed under a scanning electron microscope (SEM).

### Example 2

1 g of everolimus raw material was dispersed in 50 mL of n-heptane, added with a stir bar, stirred overnight with a stirring speed of 300 rpm at room temperature under protection against light, and then filtered to obtain a filter cake. The filter cake was vacuum dried to obtain crystalline everolimus, whose crystallinity was measured using infrared spectroscopy or powder X-ray diffraction, resulting that the crystallinity is above 90%.

7 mg of the above crystalline everolimus was put in a brown bottle, added with 10 mL of n-heptane to disperse the everolimus, and then added with 2.5 g of zirconia beads (Φ0.2-0.3 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto a surface of a balloon, and air dried for 2 hours under protection against light.

125 mg of everolimus, 25 mg of paclitaxel, and 15 mg of dibutylhydroxytoluene were dissolved in a mixed solution of 3 mL of acetone and 2 mL of ethanol, and then added with 3 mL of purified water to form a supersaturated solution of everolimus.

The prepared everolimus supersaturated solution was uniformly sprayed by ultrasonic spray onto the surface of the balloon prepared in the previous step at a drug concentration of 3 µg/mm². The rotation speed of the balloon was 200 rpm, the moving speed of the ultrasonic spray nozzle was 0.2 mm/s, and the solution ejection speed was 0.8 µL/s. After air dried for 24 hours, the balloon surface was sterilized with ethylene oxide, and the morphology thereof was observed under a scanning electron microscope (SEM). In the SEM photo, not only elongated prisms but also paclitaxel crystals on the surface of the elongated prisms can be observed.

### Example 3

1 g of rapamycin raw material was dispersed in 50 mL of n-heptane, added with a stir bar, stirred overnight with a stirring speed of 300 rpm at room temperature under protection against light, and then filtered to obtain a filter cake. The filter cake was vacuum dried at 50°C to obtain crystalline rapamycin, whose crystallinity was measured using infrared spectroscopy or powder X-ray diffraction, resulting that the crystallinity is above 90%.

A balloon catheter (with a diameter of 3.0 mm and a length of 20 mm) was provided, and 20 µL of a methanol-water solution of calcium chloride (50 mg/mL, methanol:water=1:1, v:v) was first sprayed onto the surface of the balloon catheter. 7 mg of the above crystalline rapamycin was put in a brown bottle, added with 10 mL of n-heptane to disperse the rapamycin, and then added with 2.5 g of zirconia beads (Φ0.1-0.8 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto the surface of the balloon, and air dried for 2 hours under protection against light.

125 mg of rapamycin and 15 mg of dibutylhydroxytoluene were dissolved in a mixed solution of 3 mL of acetone and 2 mL of ethanol, and then added with 3 mL of purified water to form a supersaturated solution of rapamycin.

The prepared rapamycin supersaturated solution was uniformly sprayed by ultrasonic spray onto the surface of the balloon prepared in the previous step at a drug concentration of 3 µg/mm². The rotation speed of the balloon was 200 rpm, the moving speed of the ultrasonic spray nozzle was 0.2 mm/s, and the solution ejection speed was 0.8 µL/s. After air dried for 24 hours, the balloon surface was sterilized with ethylene oxide, and the morphology thereof was observed under a scanning electron microscope (SEM).

### Example 4

Effects of supersaturated solutions of rapamycin at different concentrations on the morphology.

1 g of rapamycin raw material was dispersed in 50 mL of n-heptane, added with a stir bar, stirred overnight with a stirring speed of 300 rpm at room temperature under protection against light, and then filtered to obtain a filter cake. The filter cake was vacuum dried to obtain crystalline rapamycin, whose crystallinity was measured using infrared spectroscopy or powder X-ray diffraction, resulting that the crystallinity is above 90%.

7 mg of the above crystalline rapamycin was put in a brown bottle, added with 10 mL of n-heptane to disperse the rapamycin, and then added with 2.5 g of zirconia beads (Φ0.2-0.3 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto a surface of a balloon, and air dried for 2 hours under protection against light.

360 mg of rapamycin and 30 mg of dibutylhydroxytoluene were dissolved in a mixed solution of 3 mL of acetone and 2 mL of ethanol, and then added with 1 mL of purified water to form a supersaturated solution of rapamycin (60 mg/mL).

The prepared rapamycin supersaturated solution was uniformly sprayed by ultrasonic spray onto the surface of the balloon prepared in the previous step at a drug concentration of 3 µg/mm². The rotation speed of the balloon was 200 rpm, the moving speed of the ultrasonic spray nozzle was 0.2 mm/s, and the solution ejection speed was 0.8 µL/s. After air dried for 24 hours, the balloon surface was sterilized with ethylene oxide, and the morphology thereof was observed under a scanning electron microscope (SEM).

### Example 5

1 g of rapamycin raw material was dispersed in 50 mL of n-heptane, added with a stir bar, stirred overnight with a stirring speed of 300 rpm at room temperature under protection against light, and then filtered to obtain a filter cake. The filter cake was vacuum dried to obtain crystalline rapamycin, whose crystallinity was measured using infrared spectroscopy or powder X-ray diffraction, resulting that the crystallinity is above 90%.

7 mg of the above crystalline rapamycin was put in a brown bottle, added with 10 mL of n-heptane to disperse rapamycin, and then added with 2.5 g of zirconia beads (Φ0.2-0.3 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto a surface of a balloon, and air dried for 2 hours under protection against light.

40 mg of rapamycin and 15 mg of dibutylhydroxytoluene were dissolved in a mixed solution of 3 mL of acetone and 2 mL of ethanol, and then added with 3 mL of purified water to form a supersaturated solution of rapamycin.

The prepared rapamycin supersaturated solution was uniformly sprayed by ultrasonic spray onto the surface of the balloon prepared in the previous step at a drug concentration of 3 µg/mm². The rotation speed of the balloon was 200 rpm, the moving speed of the ultrasonic spray nozzle was 0.2 mm/s, and the solution ejection speed was 0.8 µL/s. After air dried for 24 hours, the balloon surface was sterilized with ethylene oxide, and the morphology thereof was observed under a scanning electron microscope (SEM).

### Example 6

7 mg of clarithromycin was put in a brown bottle, added with 10 mL of n-heptane to disperse the clarithromycin, and then added with 2.5 g of zirconia beads (Φ0.2-0.3 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto a surface of a balloon, and air dried for 2 hours under protection against light.

80 mg of clarithromycin and 15 mg of dibutylhydroxytoluene were dissolved in a mixed solution of 3 mL of acetone and 2 mL of ethanol, and then added with 3 mL of purified water to form a supersaturated solution of clarithromycin.

The prepared clarithromycin supersaturated solution was uniformly sprayed by ultrasonic spray onto the surface of the balloon prepared in the previous step at a drug concentration of 3 µg/mm². The rotation speed of the balloon was 200 rpm, the moving speed of the ultrasonic spraying nozzle was 0.2 mm/s, and the solution ejection speed was 0.8 µL/s. After air dried for 24 hours, the balloon surface was sterilized with ethylene oxide, and the morphology thereof was observed under a scanning electron microscope (SEM).

### Example 7

1 g of rapamycin raw material was dispersed in 50 mL of n-heptane, added with a stir bar, stirred overnight with a stirring speed of 300 rpm at room temperature under protection against light, and then filtered to obtain a filter cake. The filter cake was vacuum dried to obtain crystalline rapamycin, whose crystallinity was measured using infrared spectroscopy or powder X-ray diffraction, resulting that the crystallinity is above 90%.

7 mg of the above crystalline rapamycin was put in a brown bottle, added with 10 mL of n-heptane to disperse the rapamycin, and then added with 2.5 g of zirconia beads (Φ0.2-0.3 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto a surface of a balloon, and air dried for 2 hours under protection against light.

160 mg of rapamycin and 15 mg of dibutylhydroxytoluene were dissolved in a mixed solution of 3 mL of acetone and 2 mL of ethanol, and then added with 3 mL of purified water to form a supersaturated solution of rapamycin.

The prepared rapamycin supersaturated solution was uniformly sprayed by ultrasonic spray onto the surface of the balloon prepared in the previous step at a drug concentration of 3 µg/mm². The rotation speed of the balloon was 200 rpm, the moving speed of the ultrasonic spray nozzle was 0.2 mm/s, and the solution ejection speed was 0.8 µL/s. After air dried for 24 hours, the balloon surface was sterilized with ethylene oxide, and the morphology thereof was observed under a scanning electron microscope (SEM).

### Example 8

1 g of rapamycin raw material was dispersed in 50 mL of n-heptane, added with a stir bar, stirred overnight with a stirring speed of 300 rpm at room temperature under protection against light, and then filtered to obtain a filter cake. The filter cake was vacuum dried at 50°C to obtain crystalline rapamycin, whose crystallinity was measured using infrared spectroscopy or powder X-ray diffraction, resulting that the crystallinity is above 90%.

A balloon catheter (with a diameter of 3.0 mm and a length of 20 mm) was provided, and 20 µL of butylated hydroxytoluene (dissolved in n-heptane, 50 mg/mL) solution was first sprayed onto the surface of the balloon catheter. 7 mg of the above crystalline rapamycin was put in a brown bottle, added with 10 mL of n-heptane to disperse the rapamycin, and then added with 2.5 g of zirconia beads (Φ0.1-0.8 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto the surface of the balloon, and air dried for 2 hours under protection against light.

125 mg of rapamycin and 15 mg of dibutylhydroxytoluene were dissolved in a mixed solution of 3 mL of acetone and 2 mL of ethanol, and then added with 3 mL of purified water to form a supersaturated solution of rapamycin. The prepared rapamycin supersaturated solution was uniformly sprayed by ultrasonic spray onto the surface of the balloon prepared in the previous step at a drug concentration of 3 µg/mm². The rotation speed of the balloon was 200 rpm, the moving speed of the ultrasonic spray nozzle was 0.2 mm/s, and the solution ejection speed was 0.8 µL/s. After air dried for 24 hours, the balloon surface was sterilized with ethylene oxide, and the morphology thereof was observed under a scanning electron microscope (SEM).

### Example 9

1 g of rapamycin raw material was dispersed in 50 mL of n-heptane, added with a stir bar, stirred overnight with a stirring speed of 300 rpm at room temperature under protection against light, and then filtered to obtain a filter cake. The filter cake was vacuum dried at 50°C to obtain crystalline rapamycin, whose crystallinity was measured using infrared spectroscopy or powder X-ray diffraction, resulting that the crystallinity is above 90%.

A balloon catheter (with a diameter of 3.0 mm and a length of 20 mm) was provided. 7 mg of the above crystalline rapamycin was put in a brown bottle, added with 10 mL of n-heptane to disperse the rapamycin, and then added with 2.5 g of zirconia beads (Φ0.1-0.8 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto a surface of the balloon, and air dried for 2 hours under protection against light.

125 mg of rapamycin, 25 mg of paclitaxel, and 15 mg of dibutylhydroxytoluene were dissolved in a mixed solution of 3 mL of acetone and 2 mL of ethanol, and then added with 3 mL of purified water to form a supersaturated solution of rapamycin. The prepared supersaturated solution was uniformly sprayed by ultrasonic spray onto the surface of the balloon prepared in the previous step at a drug concentration of 3 µg/mm². The rotation speed of the balloon was 200 rpm, the moving speed of the ultrasonic spray nozzle was 0.2 mm/s, and the solution ejection speed was 0.8 µL/s. After air dried for 24 hours, the balloon surface was sterilized with ethylene oxide, and the morphology thereof was observed under a scanning electron microscope (SEM).

### Example 10

1 g of rapamycin raw material was dispersed in 50 mL of n-heptane, added with a stir bar, stirred overnight with a stirring speed of 300 rpm at room temperature under protection against light, and then filtered to obtain a filter cake. The filter cake was vacuum dried at 50°C to obtain crystalline rapamycin, whose crystallinity was measured using infrared spectroscopy or powder X-ray diffraction, resulting that the crystallinity is above 90%.

A metal stent (with a diameter of 3.0 mm and a length of 18 mm) was provided. 7 mg of the above crystalline rapamycin was put in a brown bottle, added with 10 mL of n-heptane to disperse the rapamycin, and then added with 2.5 g of zirconia beads (Φ0.1-0.8 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto a surface of the stent, and air dried for 2 hours under protection against light.

125 mg of rapamycin and 15 mg of dibutylhydroxytoluene were dissolved in a mixed solution of 3 mL of acetone and 2 mL of ethanol, and then added with 3 mL of purified water to form a supersaturated solution of rapamycin. The prepared supersaturated solution was uniformly sprayed by ultrasonic spray onto the surface of the metal stent prepared in the previous step to form a drug content of 100 µg. The rotation speed of the stent was 200 rpm, the moving speed of the ultrasonic spray nozzle was 0.2 mm/s, and the solution ejection speed was 0.8 µL/s. After air dried for 24 hours, the stent surface was sterilized with ethylene oxide, and the morphology thereof was observed under a scanning electron microscope (SEM).

### Example 11

1 g of rapamycin raw material was dispersed in 50 mL of n-heptane, added with a stir bar, stirred overnight with a stirring speed of 300 rpm at room temperature under protection against light, and then filtered to obtain a filter cake. The filter cake was vacuum dried at 50°C to obtain crystalline rapamycin, whose crystallinity was measured using infrared spectroscopy or powder X-ray diffraction, resulting that the crystallinity is above 90%.

A balloon catheter (with a diameter of 3.0 mm and a length of 20 mm) was provided. 2 mg of the above crystalline rapamycin was put in a brown bottle, added with 10 mL of n-heptane to disperse the rapamycin, and then added with 2.5 g of zirconia beads (Φ0.1-0.8 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto a surface of the balloon, and air dried for 2 hours under protection against light.

125 mg of rapamycin and 15 mg of dibutylhydroxytoluene were dissolved in a mixed solution of 3 mL of acetone and 2 mL of ethanol, and then added with 3 mL of purified water to form a supersaturated solution of rapamycin. The prepared supersaturated solution was uniformly sprayed by ultrasonic spray onto the surface of the balloon prepared in the previous step at a drug concentration of 3 µg/mm². The rotation speed of the balloon was 200 rpm, the moving speed of the ultrasonic spray nozzle was 0.2 mm/s, and the solution ejection speed was 0.4 µL/s. After air dried for 24 hours, the balloon surface was sterilized with ethylene oxide, and the morphology thereof was observed under a scanning electron microscope (SEM).

### Example 12

1 g of rapamycin raw material was dispersed in 50 mL of n-heptane, added with a stir bar, stirred overnight with a stirring speed of 300 rpm at room temperature under protection against light, and then filtered to obtain a filter cake. The filter cake was vacuum dried at 50°C to obtain crystalline rapamycin, whose crystallinity was measured using infrared spectroscopy or powder X-ray diffraction, resulting that the crystallinity is above 90%.

A balloon catheter (with a diameter of 3.0 mm and a length of 20 mm) was provided. 2 mg of the above crystalline rapamycin was put in a brown bottle, added with 10 mL of n-heptane to disperse rapamycin, and then added with 2.5 g of zirconia beads (Φ0.1-0.8 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto a surface of the balloon, and air dried for 2 hours under protection against light. Then, 20 µL of sodium benzoate water solution (15 mg/ml) was evenly sprayed onto the surface of the balloon, and air dried for 5 hours under protection against light.

125 mg of rapamycin and 15 mg of dibutylhydroxytoluene were dissolved in a mixed solution of 3 mL of acetone and 2 mL of ethanol, and then added with 3 mL of purified water to form a supersaturated solution of rapamycin. The prepared supersaturated solution was uniformly sprayed by ultrasonic spray onto the surface of the balloon prepared in the previous step at a drug concentration of 3 µg/mm². The rotation speed of the balloon was 200 rpm, and the moving speed of the ultrasonic spray nozzle was 0.2 mm/s, and the solution ejection speed was 0.4 µL/s. After air dried for 24 hours, the balloon surface was sterilized with ethylene oxide, and the morphology thereof was observed under a scanning electron microscope (SEM).

### Comparative Example 1 (positive control)

7 mg of paclitaxel was put in a brown bottle, added with 10 mL of n-heptane to disperse the paclitaxel, then added with 2.5 g of zirconia beads (Φ0.2-0.3 mm). The bottle was sealed and agitated on a vortex mixer for 10 minutes at a speed of 2500 rpm. After the agitation, the mixture was ultrasonicated in a water bath for 3 minutes, and filtered with a 40 µm nylon filter to remove the zirconia beads and obtain the filtrate. 20 µL of the filtrate was drawn by a micro syringe and evenly coated onto a surface of a balloon, and air dried for 2 hours under protection against light.

125 mg of paclitaxel and 15 mg of dibutylhydroxytoluene were dissolved in a mixed solution of 3 mL of acetone and 2 mL of ethanol, and then added with 3 mL of purified water to form a supersaturated solution of paclitaxel.

The prepared paclitaxel supersaturated solution was uniformly sprayed by ultrasonic spray onto the surface of the balloon prepared in the previous step at a drug concentration of 3 µg/mm². The rotation speed of the balloon was 200 rpm, the moving speed of the ultrasonic spray nozzle was 0.2 mm/s, and the solution ejection speed was 0.8 µL/s. After air dried for 24 hours, the balloon surface was sterilized with ethylene oxide, and the morphology thereof was observed under a scanning electron microscope (SEM).

### Comparative Example 2

125 mg of rapamycin was dissolved in a mixed solution of 3 mL of acetone and 2 mL of ethanol, and then added with 3 mL of purified water to form a supersaturated solution of rapamycin.

The prepared rapamycin supersaturated solution was uniformly sprayed by ultrasonic spray onto the surface of the balloon prepared in the previous step at a drug concentration of 3 µg/mm². The rotation speed of the balloon was 200 rpm, the moving speed of the ultrasonic spray nozzle was 0.2 mm/s, and the solution ejection speed was 0.8 µL/s. After air dried for 24 hours, the balloon surface was sterilized with ethylene oxide, and the morphology thereof was observed under a scanning electron microscope (SEM).

### Test Methods and Results

### 1. Test for Retention Time on Tissue

A group of healthy pigs weighting between 35kg to 40kg were selected and administered with aspirin at a dose of 100 mg/day and with clopidogrel at a dose of 75 mg/day for 3 days before operation. The drug balloon or drug stent prepared in the above examples were delivered to a target blood vessel segment, selected from blood vessel segments in the left circumflex (LCX) artery, left anterior descending (LAD) artery, and right coronary artery (RCA) of the coronary arteries of the pigs, dilated for 1 minute with a dilation ratio (ratio of balloon dilation diameter to blood vessel diameter) of 1.1 to 1.2, followed by a 60-second dilation period. Subsequently, the balloon or stent was deflated and withdrawn from the body. The sampling time points were set as 30 minutes, 7 days, and 28 days after the implantation, with 3 pigs used for each time point in the experiment. Postoperatively, the pigs were administered with the anticoagulant drug, clopidogrel (75mg/d) and aspirin (100mg/d). The pigs were sacrificed according to the designated time points, and tissue samples were collected for analysis of drug concentrations using high-performance liquid chromatography-mass spectrometry (HPLC-MS). The drug residues on the balloon surface after the experiment were measured using high-performance liquid chromatography (HPLC).

### 2. Firmness Test

A heart and vascular model was prepared (according to ASTM F2394-07). The drug balloon prepared in the above example was introduced into a guiding catheter along a guide wire and finally delivered to the edge of the heart model. The delivery time was controlled to be 2 minutes, and then, the balloon without dilation was withdrawn out and the residual amount of drug on the balloon was measured. The firmness was measured by the delivery loss rate of drug during the delivery to the heart and vascular model (delivery loss % = 1 - residual drug amount on the balloon/nominal drug amount, %). The lower the delivery loss rate, the better the coating firmness.

### 3. Measurement of Aspect Ratio of Drug Crystalline Particles

The drug crystalline particle carrier device prepared in each example were examined using a scanning electron microscope (SEM) to capture images of the drug coating. Then, the length and width of the drug crystalline particles were measured by using Image J software. The aspect ratio which was equal to length/width was calculated.

**Table 1. The residual amounts of drug on the surface of the drug-coated balloons of different examples**

| | Residual amount of drug on the surface of the balloon (%) |
|---|---|
| Example 1 | 6% |
| Example 2 | 5% |
| Example 3 | 5% |
| Example 4 | 6% |
| Example 5 | 8% |
| Example 6 | 4% |
| Example 7 | 5% |
| Example 8 | 5% |
| Example 9 | 7% |
| Example 10 | 6% |
| Example 11 | 9% |
| Example 12 | 8% |
| Comparative Example 1 | 12% |
| Comparative Example 2 | 8% |

**Table 2. Drug concentrations in tissue cultured for different periods with the drug-coated balloons of different examples**

| | Drug concentration in tissue (ng/mg) | | |
|---|---|---|---|
| | 30 minutes | 7 days | 28 days |
| Example 1 | 216±22 | 26±12 | 16±3.2 |
| Example 2 | 258.1±18.7 | 30.8±12.7 | 18.6±9.3 |
| Example 3 | 282.4±50.5 | 41±9 | 22±4.9 |
| Example 4 | 312±23.1 | 87.1±17.6 | 33.5±10.4 |
| Example 5 | 258.2±65 | 35.2±10.6 | 10.2±3.3 |
| Example 6 | 291±88.9 | 57±11 | 11.2±2.3 |
| Example 7 | 278±54.2 | 29.1±9.7 | 23±6.1 |
| Example 8 | 387±41.3 | 83±22.6 | 38.4±4.2 |
| Example 9 | 362±72 | 70.4±15.6 | 30.1±9.7 |
| Example 10 | 280±66.1 | 47.6±20.1 | 14±7.9 |
| Example 11 | 402±30 | 101.7±16.2 | 28.5±11 |
| Example 12 | 463±43 | 155.7±11.4 | 41.7±18.6 |
| Comparative Example 1 | 109±12 ng/mg | 17±9.1 | 1.2±0.6 |
| Comparative Example 2 | 18±2.2 ng/mg | 0.5±0.2 | 0.07±0.02 |

**Table 3. The crystal parameters and firmness of the drug-coated balloons of different examples under scanning electron microscope**

| Example | Loss Rate | Morphology of Crystal | Maximum Aspect Ratio Range | Maximum Length |
|---|---|---|---|---|
| Example 1 | 8% | Elongated prisms | 3-7 | 5 µm |
| Example 2 | 15% | Elongated prisms | 3-6 | 7 µm |
| Example 3 | 6% | Elongated prisms | 2-7 | 6 µm |
| Example 4 | 24% | Elongated prisms | 8-15 | 15 µm |
| Example 5 | 17% | Elongated prisms | 3-9 | 10 µm |
| Example 6 | 7% | Elongated prisms | 3-7 | 8 µm |
| Example 7 | 12% | Elongated prisms | 2-8 | 7 µm |
| Example 8 | 10% | Elongated prisms | 3-7 | 8 µm |
| Example 9 | 8% | Elongated prisms | 4-10 | 3 µm |
| Example 10 | 7% | Elongated prisms | 5-10 | 8 µm |
| Example 11 | 8% | Clusters of elongated prisms | 4-9 | 8 µm |
| Example 12 | 13% | Elongated prisms | 9-16 | 9 µm |
| Comparative Example 1 | 38% | Needles | 60 | 28 µm |
| Comparative Example 2 | 30% | Grains | 1 | 10 µm |

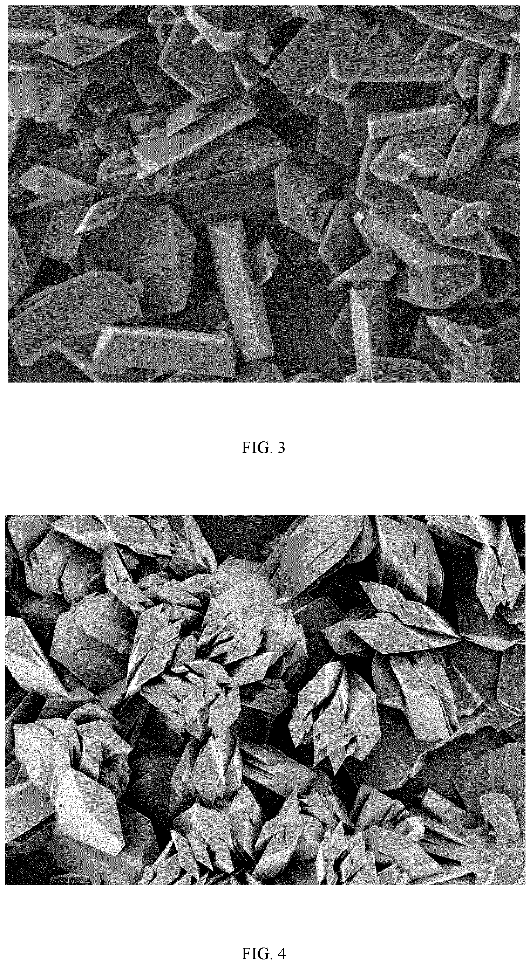

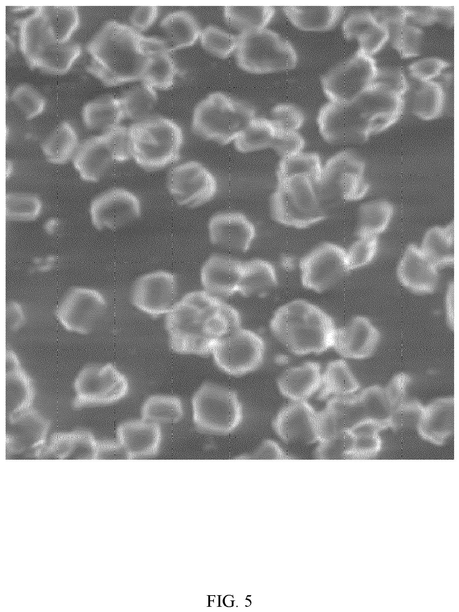

As shown in FIG. 5 and Tables 1 to 3, the prepared crystalline particles in Comparative Example 1 exhibit a more rounded shape, and the aspect ratio thereof is close to 1. In Examples 1 and 2, the surfaces of the balloons were respectively provided with rapamycin and everolimus crystal nuclei whose sizes were at the nanometer level, averaging around 500 nm, due to the agitation and grinding with extremely small zirconia beads. The subsequently sprayed supersaturated solution can grow and fuse the extremely small-sized rapamycin or everolimus crystal nuclei to form the elongated prismatic crystals. It can be seen under the electron microscope that the crystals prepared in Example 1 are elongated prisms with sharp edges and corners, which enhance the contact force with the blood vessel wall and promote drug transfer. In addition, the sharp edges and corners of the micron-sized crystalline particles of drug can effectively penetrate the tunica intima, greatly improving the efficiency of drug transfer to the vessel wall and the initial drug concentration, as well as accelerating drug transfer to the tunica media of the blood vessel, reducing drug loss caused by blood flushing. With respect to the active drug prepared in Example 4, the supersaturated solution at a high concentration can increase the aspect ratio of the active drug crystalline particles, reaching up to 10. This is possibly because the driving force for nucleation and precipitation in the supersaturated solution with the high concentration of rapamycin is stronger, leading to faster nucleation and forming more crystal nuclei. The crystal nuclei are eventually fused to form the elongated prismatic rapamycin crystals with a larger aspect ratio. In Example 12, due to the addition of the crystallization nucleating agent, the energy barrier for active nucleation are reduced, significantly reducing the nucleation rate, and finally forming the elongated prismatic crystals of rapamycin with a larger aspect ratio. It can be seen that the concentrations in tissue of Examples 1 to 12 are generally better than that of Comparative Example 2, indicating that the elongated prismatic crystals promote the immediate drug transfer, while greatly prolonging the retention of the crystallized drug in the tissue, resulting high concentrations in tissue even after 28 days. In addition, the concentrations in tissue of Example 3 and Examples 8 to 9 are higher, indicating that the layer of drug crystalline particles formed on the surface of the balloon enhances the contact force between the drug crystals and the blood vessel wall and promotes the drug transfer. Further, as the initial amount of crystal nuclei and the spraying speed are reduced, in order to achieve the set drug dose, a longer spraying time is required. With prolonged time, the rapamycin crystals gradually fuse together to form crystal clusters (see FIG. 4). These crystal clusters have multiple edges and corners and an appropriate aspect ratio, which can enhance the interaction force with the vessel wall, making it easier to penetrate the vessel wall. As the rapamycin crystal clusters can be more firmly transferred to the vessel wall, the blood flushing effect is reduced, so that the concentration in tissue is maintained for a longer time.

It can be seen from Table 3 that the drug balloons prepared in Examples 1 to 12 exhibit excellent coating firmness, with delivery losses less than or equal to 24%. In contrast, the delivery losses of Comparative Examples 1 and 2 are more than 30%, the firmness is poor, making the drug easy to loss during delivery. The drug balloons prepared in Examples 1 to 12 of the present application have excellent firmness and can be used in complex lesions. This is likely due to that the technical solution in the present application abandons the hydrophilic excipients commonly used in traditional coated balloons. Furthermore, the prismatic morphology of the drug crystals results in greater contact force with the device substrate, significantly reducing drug loss in delivery.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present application.

The above-described embodiments are only several implementations of the present application, and the descriptions are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the patent protection of the present application shall be defined by the appended claims, the description and drawings may be used to illustrate the content of the claim.

## Claims

1. A drug-loaded medical device, including a medical device body and at least one drug coating, the drug coating is attached to at least one side of a surface of the medical device body,
wherein a main component of the drug coating comprises a macrolide drug, the drug coating comprises elongated prismatic crystals each with at least three lateral edges, and/or the drug coating includes crystal clusters of the elongated prismatic crystals each with at least three lateral edges, and an aspect ratio of each elongated prismatic crystal is in a range from 1: 1 to 40: 1.

2. The drug-loaded medical device according to claim 1, wherein each elongated prismatic crystal comprises at least two adjacent lateral surfaces with an included angle smaller than 90 degrees.

3. The drug-loaded medical device according to claim 1, wherein a length of each elongated prismatic crystal is smaller than 25 µm.

4. The drug-loaded medical device according to claim 1, wherein a loss rate of the drug coating attached to the surface of the medical device body is less than or equal to 24%.

5. The drug-loaded medical device according to claim 1, wherein the macrolide drug is selected from the group consisting of rapamycin, zotarolimus, everolimus, temsirolimus, biolimus, 7-O-desmethylrapamycin, temsirolimus, ridaforolimus, 40-O-(2-hydroxyethyl)-rapamycin, 32-deoxo rapamycin, biolimus A9, ABT-578, SDZ RAD, SAR943, and combinations thereof.

6. The drug-loaded medical device according to claim 1, wherein the drug coating further comprises at least one of a) to d):
a) a drug delivery carrier layer disposed on either an upper base or a lower base of each elongated prismatic crystal, and a main component of the drug delivery carrier layer is selected from the group consisting of calcium chloride, polyvinylpyrrolidone, polyvinyl alcohol, collagen, gelatin, polysaccharide, stearic acid, iopromide, iopamidol, iohexol, ioversol, urea, citric acid, glucan, polysorbate, sorbitol, chitosan, poloxamer, sodium carboxymethylcellulose, hydroxypropyl methylcellulose, sodium hyaluronate, sodium alginate, ammonium shellacate, n-butyryl tri-n-hexyl citrate, shellac, PEG, and combinations thereof;
b) an antioxidant, selected from the group consisting of dibutyl hydroxytoluene, butylated hydroxyanisole, ascorbic acid, palmitate, tocopherol, probucol, vitamin E, polyethylene glycol succinate, and combinations thereof;
c) an organic crystallization nucleating agent, selected from the group consisting of a salt of an organic acid, a dibenzylidene sorbitol derivative, a diamide, a hydrazide, and combinations thereof;
d) at least one active drug other than the macrolide drug, selected from the group consisting of paclitaxel, a paclitaxel derivative, cilostazol, ticlopidine, a phosphodiesterase inhibitor, nafronyl, pentoxifylline, rivaroxaban, apixaban, dabigatran etexilate, tirofiban, a thrombin inhibitor, a factor Xa inhibitor, a vitamin K inhibitor, a cyclooxygenase inhibitor, an ADP receptor antagonist or inhibitor, a platelet glycoprotein IIb/IIIa receptor antagonist, dexamethasone, prednisolone, corticosterone, budesonide, a natriuretic peptide, an estrogen, sulfasalazine, aminosalicylic acid, acemetacin, aescin, aminopterin, antimycoin, arsenic trioxide, aristolochic acid, aspirin, berberine, bilobol, endostatin, angiostatin, angiopeptin, an monoclonal antibody that can block the proliferation of smooth muscle cells, levofloxacin, hydroxycamptothecin, vinblastine, vincristine, doxorubicin, 5-fluorouracil, cisplatin, a bisphosphonate, a selective estrogen receptor modulator, strontium ranelate, actinomycin D, cyclosporine A, cyclosporine C, brefeldin A, relaxin, a guanylate activating enzyme regulator, nitroxyl prodrug CXL-1020, a nitrate drug, sodium nitroprusside, nitroglycerin, a thymidine kinase inhibitor antibiotic, nesiritide, a calcium channel blocker such as diltiazem, verapamil, nicardipine, platelet vasodilator-stimulated phosphoprotein, vasodilator-stimulated phosphoprotein, papaverine, adenosine, magnesium sulfate, xantinol nicotinate, nicorandil, and combinations thereof;
the paclitaxel derivative is selected from the group consisting of docetaxel, nab-paclitaxel, nap-paclitaxel, and combinations thereof.

7. A method for preparing a drug-loaded medical device, comprising:
1) dispersing macrolide drug crystal powder in a solvent, adding rigid microspheres, agitating, and filtering out the rigid microspheres to obtain a suspension;
2) taking a medical device body, applying the suspension onto a surface of the medical device body, and drying the surface to obtain a medical device intermediate; and
3) applying a supersaturated solution of the macrolide drug onto a surface of the medical device intermediate and drying the surface.

8. The method according to claim 7, wherein the suspension comprises crystalline microparticles of the macrolide drug with a particle size in a range from 100 nm to 1000 nm.

9. The method according to claim 8, wherein a concentration of the supersaturated solution is in a range from 1 mg/mL to 100 mg/mL.

10. The method according to any one of claims 7 to 9, wherein the solvent is selected from the group consisting n-heptane, n-hexane, pentane, cyclohexane, and combinations thereof.

11. The method according to claim 8 or 9, wherein the rigid microspheres are made of a material selected from the group consisting of zirconium oxide, silicon nitride, hard stainless steel, hard tungsten carbide, sintered corundum, agate, and combinations thereof, and the particle size of the rigid microspheres is 800 to 1200 times of the particle size of the crystalline microparticles.

12. The method according to claim 7, wherein in step 2), a drug delivery carrier layer is disposed on the surface of the medical device body, and a main component of the drug delivery carrier layer is selected from the group consisting of calcium chloride, polyvinylpyrrolidone, polyvinyl alcohol, collagen, gelatin, polysaccharide, stearic acid, iopromide, iopamidol, iohexol, ioversol, urea, citric acid, glucan, polysorbate, sorbitol, chitosan, poloxamer, sodium carboxymethylcellulose, hydroxypropyl methylcellulose, sodium hyaluronate, sodium alginate, ammonium shellacate, n-butyryl tri-n-hexyl citrate, shellac, PEG, and combinations thereof.

13. The method according to claim 7, further comprising spraying a solution of an organic crystallization nucleating agent onto the surface of the medical device intermediate and drying the surface, wherein the organic crystallization nucleating agent is selected from the group consisting of a salt of an organic acid, a dibenzylidene sorbitol derivative, a diamide, a hydrazide, and combinations thereof.

14. The method according to claim 7, wherein the supersaturated solution of the macrolide drug further comprises an antioxidant and/or at least one active drug other than the macrolide drug;
the antioxidant is selected from the group consisting of dibutyl hydroxytoluene, butylated hydroxyanisole, ascorbic acid, palmitate, tocopherol, probucol, vitamin E, polyethylene glycol succinate, and combinations thereof;
the at least one active drug other than the macrolide drug is selected from the group consisting of paclitaxel, a paclitaxel derivative, cilostazol, ticlopidine, a phosphodiesterase inhibitor, nafronyl, pentoxifylline, rivaroxaban, apixaban, dabigatran etexilate, tirofiban, a thrombin inhibitor, a factor Xa inhibitor, a vitamin K inhibitor, a cyclooxygenase inhibitor, an ADP receptor antagonist or inhibitor, a platelet glycoprotein IIb/IIIa receptor antagonist, dexamethasone, prednisolone, corticosterone, budesonide, a natriuretic peptide, an estrogen, sulfasalazine, aminosalicylic acid, acemetacin, aescin, aminopterin, antimycoin, arsenic trioxide, aristolochic acid, aspirin, berberine, bilobol, endostatin, angiostatin, angiopeptin, an monoclonal antibody that can block the proliferation of smooth muscle cells, levofloxacin, hydroxycamptothecin, vinblastine, vincristine, doxorubicin, 5-fluorouracil, cisplatin, a bisphosphonate, a selective estrogen receptor modulator, strontium ranelate, actinomycin D, cyclosporine A, cyclosporine C, brefeldin A, relaxin, a guanylate activating enzyme regulator, nitroxyl prodrug CXL-1020, a nitrate drug, sodium nitroprusside, nitroglycerin, a thymidine kinase inhibitor antibiotic, nesiritide, a calcium channel blocker such as diltiazem, verapamil, nicardipine, platelet vasodilator-stimulated phosphoprotein, vasodilator-stimulated phosphoprotein, papaverine, adenosine, magnesium sulfate, xantinol nicotinate, nicorandil, and combinations thereof;
the paclitaxel derivative is selected from the group consisting of docetaxel, nab-paclitaxel, nap-paclitaxel, and combinations thereof.
